# EUROPÄISCHE PATENTANMELDUNG

(11) **EP 1 136 091 A1**
(43) Veröffentlichungstag der Anmeldung: **26.09.2001**
(21) Anmeldenummer: 01105833.6
(22) Anmeldetag: 09.03.2001
(51) Int. Cl.: A61M 5/32

(54) **Kanüle**

(30) Priorität: 16.03.2000 DE 10012886
(71) Anmelder: TRANSCOJECT GESELLSCHAFT FÜR MEDIZINISCHE GERÄTE mbH & CO. KG, 24539 Neumünster (DE)
(72) Erfinder: Rolle, Philipp, 24539 Neumünster (DE)
(74) Vertreter: Vollmann, Heiko, Dipl.-Ing.

(57) **Zusammenfassung**

Die Kanüle ist zum Austragen von flüssigen oder pastösen Stoffen vorgesehen. Am freien Ende der Kanüle ist ein diese abschließender Verschlusskörper (3) angeformt. Im Bereich zwischen der Kanüle (1) und dem Verschlusskörper (3) ist eine Sollbruchstelle (4) vorgesehen, so dass der Verschlusskörper (3) durch einfaches Brechen von der Kanüle (1) entfernt werden kann. Der Verschlusskörper (3) kann dann zum späteren Wiederverschließen auf das Ende der Kanüle (1) aufgesetzt werden.

## Beschreibung

Die Erfindung betrifft eine Kanüle zum Austragen von flüssigen oder pastösen Stoffen.

Um flüssige oder pastöse Stoffe, wie beispielsweise zahnärztliche Füllmaterialien, Klebstoffe oder dergleichen präzise und gezielt austragen zu können, werden Kanülen eingesetzt, die auf das Ende einer Spritze aufgesetzt werden, die als Dosiergefäß dient. Das Aufsetzen der Kanüle erfolgt nach Entfernen eines Spritzenverschlusses.

Zwar hat sich der Einsatz von Kanülen zum Austragen von derartigen Stoffen bestens bewährt, doch ist es häufig so, dass nicht der gesamte Spritzeninhalt für eine Anwendung benötigt wird, sondern dass eine mehrfache Entnahme wünschenswert ist, um den gesamten Spritzeninhalt nach und nach nutzen zu können. Da es sich bei diesen Stoffen in der Regel um aushärtende oder austrocknende Stoffe handelt, muss dafür gesorgt werden, dass die Spritze nach der jeweiligen Anwendung wieder dicht verschlossen wird, das heißt, die Kanüle muss entfernt und der Verschlussstopfen auf die Spritze aufgesetzt werden. Abgesehen davon, dass das Abnehmen der Kanüle und Verschließen der Spritze verhältnismäßig umständlich ist, besteht hierbei auch die Gefahr, dass, insbesondere bei flüssigen Stoffen, Stoffreste aus der Kanüle tropfen. Im Übrigen ist für jede Anwendung eine neue Kanüle erforderlich. Ein Reinigen der Kanüle ist praktisch nicht möglich, da in der Regel der Innenquerschnitt so klein bemessen ist, dass eine Reinigung mit wirtschaftlichem Aufwand nicht sinnvoll ist.

Vor diesem Stand der Technik liegt der Erfindung die Aufgabe zugrunde, eine gattungsgemäße Kanüle so auszubilden, dass ein mehrmaliger Einsatz ermöglicht wird. Darüber hinaus soll nach Möglichkeit auch die Handhabung des gesamten Produktes, zu dem die Kanüle gehört, vereinfacht werden.

Diese Aufgabe wird gemäß der Erfindung durch die in Anspruch 1 angegebenen Merkmale gelöst. Vorteilhafte Ausgestaltungen der Erfindung sind den Unteransprüchen, der nachfolgenden Beschreibung sowie der Zeichnung zu entnehmen.

Grundgedanke der vorliegenden Erfindung ist es, eine Reinigung der Kanüle bei mehrfachem Gebrauch dadurch entbehrlich zu machen, dass diese nach dem Gebrauch dicht verschlossen wird, also gar nicht von der Spritze entfernt wird. Der Verschlusskörper selbst ist am freien Ende der Kanüle angeformt und schließt diese zunächst ab (Originalzustand). Es ist eine Sollbruchstelle im Bereich zwischen der Kanüle und dem Verschlusskörper vorgesehen, so dass der Verschlusskörper durch Handkraft von der Kanüle getrennt werden kann. Nach dem Trennen des Verschlusskörpers von der Kanüle ist diese endseitig (an ihrem distalen Ende) offen. Das erneute Verschließen erfolgt dann mittels des Verschlusskörpers, der zuvor von der Kanüle getrennt worden ist.

Die Erfindung ermöglicht nicht nur Kanüle und Verschlusskörper einstückig als Spritzgussteil aus Kunststoff oder Glas auszubilden, was fertigungstechnisch günstig ist, sondern gibt darüber hinaus auch den Vorteil, dass stets sichergestellt ist, dass nach dem Öffnen auch ein Verschlusskörpervorhanden ist. Es kann also nicht passieren, dass dem Produkt versehentlich kein Verschlusskörper beigepackt wird. Darüber hinaus hat das Anformen des Verschlusskörpers an die Kanüle den Vorteil, dass diese im Originalzustand endseitig hermetisch abgeschlossen ist.

Die vorliegende Erfindung erlaubt somit auch eine einstückige Ausbildung von Spritzenkörper und Kanüle, was hinsichtlich der Fertigungskosten von Vorteil ist.

Alternativ kann die erfindungsgemäße Kanüle auch als gesondertes Bauteil, beispielsweise mit Luer- und/oder Luer Lock-Anschluss versehen sein, so dass sie mit üblichen Spritzen eingesetzt werden kann. Die Kanüle kann dann also auch zum mehrfachen Austragen von Stoffen aus Spritzen erfolgen, wie sie zum Stand der Technik zählen und einleitend beschrieben worden sind.

Der Verschlusskörper kann entsprechend den Anforderungen ausgebildet sein, beispielsweise als Verschlusskappe, die über das freie Ende der Kanüle gestülpt wird oder auch als Stopfen, der in das freie Ende der Kanüle eingeführt wird. Auch eine Kombination beider Verschlusskörperformen ist denkbar, indem innerhalb der Verschlusskappe noch zusätzlich ein Stopfen eingeformt ist, so dass das freie Kanülenende vom Verschlusskörper allseits umgeben wird.

Besonders vorteilhaft ist es, wenn die Sollbruchstelle am Ende der Kanüle liegt, also unmittelbar vor dem Verschlusskörper, da dann einerseits die volle Kanülenlänge zum Austragen erhalten und andererseits der Verschlusskörper nach dem Entfernen von einer vorgefüllten Spritze weitgehend frei von Stoffresten bleibt.

Um sicherzustellen, dass sich der Verschlusskörper nicht versehentlich vom Kanülenende löst, ist es zweckmäßig, Rastmittel zwischen Verschlusskörper und Kanüle vorzusehen. Bei Verwendung einer Verschlusskappe kann beispielsweise innerhalb der Kappe eine umlaufende Nut vorgesehen sein, die mit entsprechend am Außenumfang der Kanüle vorgesehenen Anformungen in Eingriff bringbar ist, oder umgekehrt.

Fertigungstechnisch günstig ist es, den Verschlusskörper am Ende der Kanüle so anzuformen, dass er zum bestimmungsgemäßen erneuten Verschließen um 180° gedreht aufzusetzen ist. Dann nämlich ergibt sich ein vergleichsweise einfaches Spritzgießwerkzeug, auch können vergleichsweise kleine Lumina realisiert werden.

Um das Entfernen des Verschlusskörpers von der Kanüle zu erleichtern ist es zweckmäßig, an diesem eine Handhabe anzuformen. Eine solche Handhabe vergrößert die Grifffläche und erleichtert sowohl das erstmalige Abnehmen, also das Abbrechen des Verschlusskörpers von der Kanüle, als auch das wiederholte Auf- und Absetzen, insbesondere, wenn beispielsweise im Randbereich zwischen Verschlusskappe und Kanüle der auszutragende Stoff antrocknet oder aushärtet.

Die Erfindung ist nachfolgend anhand eines in der Zeichnung dargestellten Ausführungsbeispiels näher erläutert. Es zeigen
- Figur 1a - c: in vereinfachter schematischer Schnittdarstellung, wie die erfindungsgemäße Kanüle bestimmungsgemäß zu gebrauchen ist,
- Figur 2: in vergrößerter Schnittdarstellung das Ende der Kanüle gemäß Figur la mit Verschlusskörper,
- Figur 3: ein zweites Ausführungsbeispiel einer Kanüle im Längsschnitt und
- Figur 4: eine Ansicht in Richtung des Pfeils IV in Figur 3.

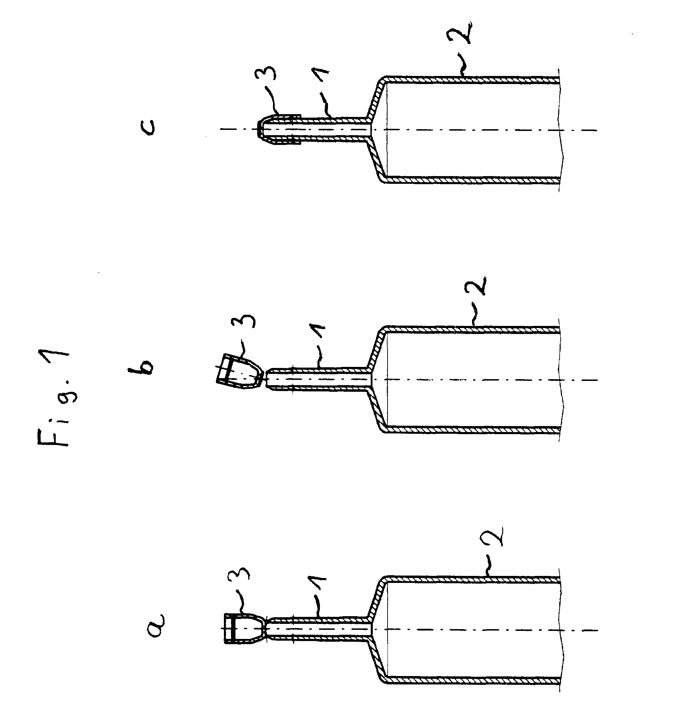

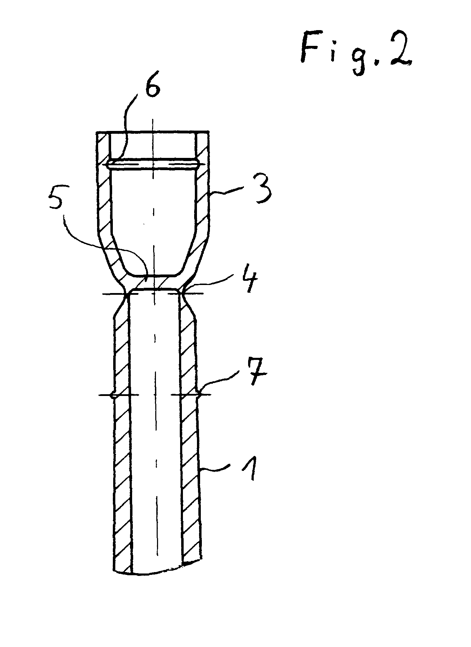

Die anhand der Figuren 1 und 2 dargestellte Kanüle 1 hat im Wesentlichen eine zylindrische Form und ist einstückig mit einem Spritzenkörper 2 ausgebildet, in den in an sich bekannter Weise ein nicht dargestellterSpritzenkolben eintaucht. Am distalen Ende der Kanüle 1 ist eine Verschlusskappe 3 angeformt, die zugleich die Kanüle 1 endseitig abschließt. Zwischen dem freien Ende der Kanüle 1 und der Verschlusskappe 3 ist eine Sollbruchstelle 4 vorgesehen, das heißt, in diesem Bereich ist die Wandung im Vergleich zur übrigen Kanüle 1 deutlich dünner ausgebildet, so dass die Verschlusskappe 3, wie in Figur 1b angedeutet, von der Kanüle 1 abgebrochen werden kann, und sich einerseits ein möglichst glatter Abschluss am Kanülenende bildet, andererseits die Kräfte zum Entfernen der Verschlusskappe 3 manuell beherrschbar sind. Die Sollbruchstelle 4 ist so gestaltet, dass sich die Kanüle 1 zum Ende hin im Außenumfang verjüngt, wohingegen das Lumen konstant bleibt. Dies hat den Vorteil, dass quasi eine zulaufende Spitze gebildete wird, welche das gezielte Austragen erleichtert.

Die Verschlusskappe 3 ist becherförmig ausgestaltet und vom Innendurchmesser so dimensioniert, dass sie auf die Kanüle 1 nach dem Entfernen umgekehrt aufsetzbar ist (siehe Figur 1 c). Dabei läuft die Außenwand der Verschlusskappe 3 zur Querwand 5 hin konisch zu um einen dichten Abschluss in diesem Bereich zu gewährleisten. Am Innenumfang der Verschlusskappe 3 ist eine im Querschnitt halbkreisförmige Nut 6 vorgesehen, die Teil einer Rastverbindung ist. Komplementär zu dieser Nut 6 ist am Außenumfang der Kanüle 1 ein Ring 7 vorgesehen, der einen ebenfalls halbkreisförmigen Querschnitt aufweist, so dass beim Aufstecken der Verschlusskappe 3 auf das Ende der Kanüle 1 (Figur 1 c) dieser Ring 7 in die Nut 6 eingreift, wodurch die Verschlusskappe 3 zuverlässig auf dem Kanülenende gehalten wird. Je nach verwendetem Material kann der Ring 7 auch durch Einzelvorsprünge gebildet oder eine andere Rastverbindung vorgesehen sein.

### Der Gebrauch der vorbeschriebenen Kanüle ergibt sich wie folgt:

Ein mit dem auszutragenden Stoff, z. B. einer zahnärztlichen Füllmasse, gefüllter Spritzenzylinder ist an seinem offenen Ende durch den Spritzenkolben verschlossen, so dass der Stoff innerhalb der Spritze hermetisch abgeschlossen ist (Figur 1a). Zum Gebrauch wird nun durch Drehen oder Knicken die Verschlusskappe 3 von der Kanüle 1 getrennt (Figur 1b). Dadurch wird die Kanüle 1 an ihrem freien Ende, also dem vom Spritzenkörper abgewandten Ende geöffnet. Durch Eindrücken des nicht dargestellten Spritzenkolbens wird der im Spritzenkörper befindliche Stoff über die Kanüle 1 ausgetragen. Die Kanüle 1 wird danach durch Aufsetzen der Verschlusskappe 3 in der in Figur 1 c dargestellten Weise wieder verschlossen. Durch die Rastverbindung zwischen Nut 6 und Ring 7 sitzt die Verschlusskappe 3 dicht und fest auf dem Ende der Kanüle 1. Zum erneuten Austragen wird dann die Verschlusskappe 3 abgenommen und nach dem Austragen wieder aufgesetzt. Der im Spritzenkörper 2 und innerhalb der Kanüle 1 befindliche Stoff ist nach dem Aufsetzen der Verschlusskappe 3 von der Außenatmosphäre abgeschlossen, so dass nicht die Gefahr besteht, dass dieser innerhalb der Kanüle aushärtet oder austrocknet. Die Kanüle kann somit wieder verwendet werden, bis der gesamte Spritzeninhalt verbraucht ist. Da bei der Ausführung nach Figureb 1 und 2 Spritzenkörper 2, Kanüle 1 und Verschlusskappe 3 einstückig als Spritzgussteil aus Kunststoff ausgebildet sind, sind die Herstellungskosten sehr günstig und im Vergleich zu einem einfachen Spritzenkörper - genügende Stückzahl vorausgesetztnur unerheblich höher.

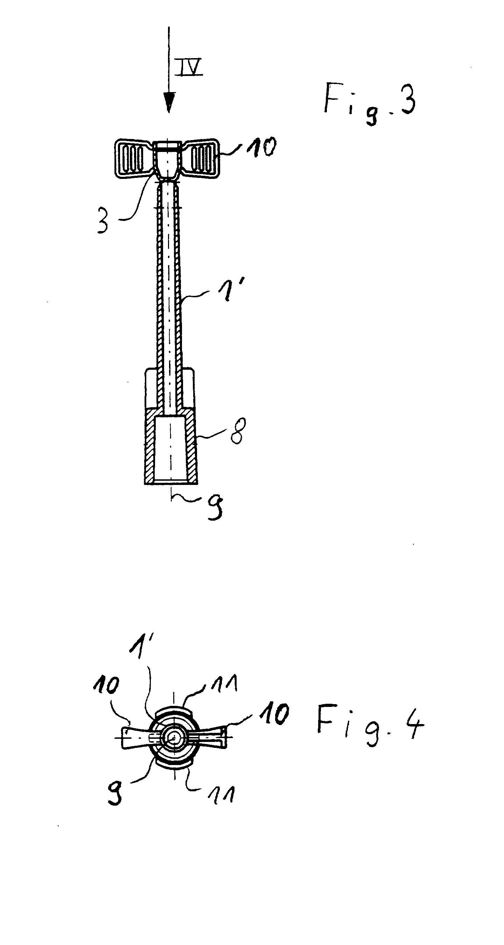

Die Ausführung nach den Figuren 3 und 4 unterscheidet sich von der vorbeschriebenen dadurch, dass die Kanüle 1' nicht einstückig mit dem Spritzenkörper 2 ausgebildet ist, sondern als gesondertes Kanülenteil, wie dies auch aus dem Stand der Technik bekannt ist. Kanüle 1' ist daher an ihrem spritzenseitigen Ende mit einem Lueranschluss 8 versehen. Im Übrigen entspricht die Ausbildung im Wesentlichen der vorbeschriebenen. Zur besseren Handhabung sind an die Verschlusskappe 3 seitlich um 180° zueinander versetzt (bezogen auf die Kanülenachsen 9) zwei Flügel 10 angeformt, die eine Handhabe bilden. Diese Flügel 10 geben guten Halt, so dass die Verschlusskappe 3 zwischen zwei Fingern und dem Daumen sicher gehalten werden kann, was insbesondere zum erstmaligen Abbrechen von der Kanüle 1' aber auch beim späteren Auf- und Absetzen von Vorteil ist.

Wie die Draufsicht nach Figur 4 erkennen läßt, ist der Lueranschluss 8 im Außenumfang mit zwei Vorsprüngen 11 versehen, so dass auch eine Verbindung mit einem Luer Lock-Anschluss möglich ist, wie dies an sich bei Kanülen aus der Medizintechnik bekannt ist.

Die Handhabung der Kanüle 1' erfolgt in analoger Weise zu der vorbeschriebenen, mit dem Unterschied, dass vor dem erstmaligen Gebrauch der Lueranschluss mit der gefüllten Spritze verbunden wird.

### Bezugszeichenliste

- 1, 1' -: Kanüle
- 2 -: Spritzenkörper
- 3 -: Verschlusskappe
- 4 -: Sollbruchstelle
- 5 -: Querwand
- 6 -: Nut
- 7 -: Ring
- 8 -: Lueranschluss
- 9 -: Kanülenachse
- 10 -: Flügel
- 11 -: Vorsprünge

## Patentansprüche

1. Kanüle zum Austragen von flüssigen oder pastösen Stoffen, **dadurch gekennzeichnet, dass** am freien Ende der Kanüle ein diese abschließender Verschlusskörper (3) angeformt ist, dass eine Sollbruchstelle (4) im Bereich zwischen der Kanüle (1, 1') und dem Verschlusskörper (3) vorgesehen ist und dass der Verschlusskörper (3) so ausgebildet ist, dass er nach dem Trennen von der Kanüle (1, 1') zum erneuten Verschließen derselben einsetzbar ist.

2. Kanüle nach Anspruch 1, **dadurch gekennzeichnet, dass** der Verschlusskörper als Kappe (3) ausgebildet ist, die nach dem Trennen von der Kanüle (1, 1') auf diese zum Zwecke des erneuten Verschließens aufsetzbar ist.

3. Kanüle nach Anspruch 1, **dadurch gekennzeichnet, dass** der Verschlusskörper stopfenartig ausgebildet und nach dem Trennen von der Kanüle in diese zum Zwecke des erneuten Verschließens einsetzbar ist.

4. Kanüle nach einem der vorhergehenden Ansprüche, **dadurch gekennzeichnet, dass** sie (1') an ihrem spritzenseitigen Ende mit einem Luer- oder Luer Lock-Anschluss (8) versehen ist.

5. Kanüle nach einem der vorhergehenden Ansprüche, **dadurch gekennzeichnet, dass** sie an das Ende einer Spritze (2) angeformt ist.

6. Kanüle nach einem der vorhergehenden Ansprüche, **dadurch gekennzeichnet, dass** eine Sollbruchstelle (4) am Ende der Kanüle (1, 1') unmittelbar vor dem Verschlusskörper (3) vorgesehen ist.

7. Kanüle nach einem der vorhergehenden Ansprüche, **dadurch gekennzeichnet, dass** Rastmittel (6, 7) zwischen Kanüle (1, 1') und Verschlusskörper (3) vorgesehen sind.

8. Kanüle nach einem der vorhergehenden Ansprüche, **dadurch gekennzeichnet, dass** der Verschlusskörper (3) so an das Kanülenende angeformt ist, dass er nach dem Trennen von der Kanüle (1, 1') zum erneuten Verschließen um 180° gedreht aufzusetzen ist.

9. Kanüle nach einem der vorhergehenden Ansprüche, **dadurch gekennzeichnet, dass** am Verschlusskörper (1') mindestens eine Handhabe (10) angeformt ist.
